# EUROPÄISCHE PATENTANMELDUNG

(11) **EP 1 101 474 A1**
(43) Veröffentlichungstag der Anmeldung: **23.05.2001**
(21) Anmeldenummer: 99125381.6
(22) Anmeldetag: 20.12.1999
(51) Int. Cl.: A61F 13/38

(54) **Endoskopisches Instrument zur Präparation**

(30) Priorität: 17.11.1999 AT 80099 U
(71) Anmelder: AMI (Agency for medical innovations GmbH), 6840 GötzisAT (AT)
(72) Erfinder: Höfle, Siegfried, Dipl.-Ing., 6840 Götzis (AT)
(74) Vertreter: Riebling, Peter, Dr.-Ing.

(57) **Zusammenfassung**

Die Erfindung betrifft ein endkoskopisches Instrument zur Präparation bei mikrochirurgischen Operationen und Untersuchungen, mit einem gesteigerten Flüssigkeits-Saugvermögen, wobei eine als Tupfer ausgebildete Mullkappe (12) am vorderen, freien Ende einer Zylinderkammer (15) angeordnet ist, und die Mullkappe auf ein Stützelement (16) aufgeformt ist. Durch den üblicher Weise im Operationsgebiet herrschenden Überdruck wird die abzuführende Flüssigkeit automatisch durch den Tupfer hindurch in die Zylinderkammer geleitet, wo ein großes Aufnahmevolumen zur Verfügung steht. Eine erste Ausgestaltung der Erfindung sieht vor, daß der Stützkörper mittels einer widerhakenartigen und bevorzugt lösbaren Verrastung in der Trennwand zwischen Zylinderkammer und Halterungsschaft einrastet. Bei einer zweiten Ausführungsform der Erfindung ist in der Zylinderkammer ein Kolben (9) abdichtend und längsverschiebbar geführt, welcher beim Zurückziehen einen zusätzlichen Unterdruck auf die am distalen Ende angeordnete Mullkappe ausübt. Das endoskopische Instrument ist leicht auswechselbar am vorderen freien Ende eines endoskopischen Applikationsrohres angeordnet, welches seinerseits fest mit einem endoskopischen Applikator verbunden ist.

## Beschreibung

Die Erfindung betrifft ein endkoskopisches Instrument zur Präparation bei mikrochirurgischen Operationen und Untersuchungen nach dem Oberbegriff des unabhängigen Anspruchs 1.

Im Gegenstand der US 5 312 333 ist ein endoskopischer Applikator bekannt geworden, bei dem in einem Applikationsrohr eine Kolbenstange verschiebbar ausgebildet ist, und vor der ein Tupferelement angeordnet ist, welches mit der Kolbenstange in das Gewebe ausgestoßen werden kann. Es handelt sich also dort nicht um einen Tupfer, der zur Aufnahme von Blut oder Gewebsflüssigkeiten geeignet ist, sondern mit diesem bekannten Tupferelement wird ein Medikament in den Körper hinein abgegeben.

Ein derartiger Applikator ist nicht geeignet, größere Mengen von Blut oder Gewebsflüssigkeit aufzunehmen, denn bei diesem besteht die Gefahr, daß der Tupfer aus dem Applikationsrohr heraus in den Körper gerät und dort verbleibt. Er ist dann nur wieder schwierig zu entfernen.

Weiterer Nachteil dieser bekannten Anordnung ist, daß der Wirkungsgrad (Aufsaugvermögen) eines derartigen Tupfers relativ gering ist.

Der Erfindung liegt deshalb die Aufgabe zugrunde, ein endoskopisches Instrument zur Präparation mit einem Tupfer so weiter zu bilden, daß er sicher und fest an einem Applikator gehalten werden kann, ohne daß die Gefahr des Verlierens besteht, und daß er einen sehr guten Wirkungsgrad bezüglich seines Saugvermögens aufweist.

Zur Lösung der gestellten Aufgabe ist die Erfindung durch die technische Lehre des Anspruches 1 gekennzeichnet.

Wesentliches Merkmal der Erfindung ist, daß eine als Tupfer ausgebildete Mullkappe am vorderen, freien Ende einer Zylinderkammer angeordnet ist.

Mit der gegebenen technischen Lehre ergibt sich also der wesentliche Vorteil, daß in ansich bekannter Weise eine Mullkappe an einem endoskopischen Instrument angeordnet ist. Erfindungsgemäß ist aber die Mullkappe am vorderen freien Ende einer Zylinderkammer angeordnet und ist dort gesichert gehalten, so daß durch die Spitze der Mullkappe hindurch Blut oder Gewebsflüssigkeit aufgesaugt werden kann.

Damit wird der Durchtränkungsgrad der Mullkappe wesentlich verbessert, denn aufgrund des Überdruckes, welcher im Operationsgebiet herrscht, in dem der Tupfer eingesetzt wird, wird sogar Gewebsflüssigkeit durch die Mullkappe hindurch in die Zylinderkammer gedrückt, wodurch ein ausgezeichneter Füllungsgrad erreicht wird. Damit kann mit einem relativ kleinen Volumen der Mullkappe ein großes Aufsaugvermögen erreicht werden.

Es hat sich nämlich herausgestellt, daß, im Operationsgebiet, in dem der Tupfer eingesetzt wird, ein Überdruck gegenüber der Atmosphäre besteht, welcher zum Füllen der Zylinderkammer durch den Tupfer hindurch ausgenutzt wird, ohne daß hierbei eine zusätzliche Druckdifferenz zwischen Tupfer und Zylinderkammer angelegt werden muß.

Hierbei wird vorausgesetzt, daß der Tupfer abgedichtet im Operationsgebiet angeordnet ist, er also pfropfenartig das Operationsgebiet gegenüber der Atmosphäre abdichtet.

In einer zweiten Ausführungsform ist in der Zylinderkammer zusätzlich ein Kolben abdichtend, längsverschiebbar geführt, wobei dieser Kolben beim Zurückziehen einen zusätzlichen Unterdruck auf die am distalen Ende angeordnete Mullkappe aus übt, zusätzlich zum bereits im Operationsgebiet herrschenden Überdruck, welcher dann zwischen Tupfer und Zylinderkammer herrscht.

Damit wird der Durchtränkungsgrad der Mullkappe noch einmal erhöht, denn aufgrund des durch den Kolben angelegten Unterdruckes kann Gewebsflüssigkeit durch die Mullkappe hindurch in die Zylinderkammer eingesaugt werden, wodurch ein nochmal verbesserter Füllungsgrad erreicht werden kann. Damit kann mit einem relativ kleinen Volumen der Mullkappe ein noch größeres Aufsaugvermögen erreicht werden.

Ein derartiger Tupfer dient als Präpariertupfer. Er dient insbesondere zur Präparation, z. B. im Bereich des Schallot'schen Dreiecks und zum Aufsaugen von Blut oder Gewebsflüssigkeit in schwierigsten Situationen, wenn ein Absaugen direkt nicht durchführbar ist.

Wichtig ist bei der Erfindung, daß das damit geschaffene endoskopische Instrument leicht auswechselbar am vorderen freien Ende eines endoskopischen Applikationsrohres angeordnet ist, welches seinerseits fest mit einem endoskopischen Applikator verbunden ist. Die Befestigung erfolgt bevorzugt leicht lösbar z. B. mit Hilfe einer Rastbefestigung. Statt dieser Rastbefestigung können jedoch auch andere leicht lösbare Befestigungen verwendet werden, wie z. B. eine Schraubverbindung oder eine Bajonettsteckverbindung.

Zur sicheren Halterung der Mullkappe in der Zylinderkammer ist es in einer Weiterbildung vorgesehen, daß die Mullkappe auf ein Stützelement aufgeformt ist, welches Stützelement ein oder mehrere in axialer Richtung sich erweiternde Vorsprünge aufweist, um ein herausziehen der Mullkappe von diesem Stützelement zu vermeiden.

Bevorzugt ist das Stützelement an seinem distalen Ende mit einem Kopf ausgebildet, der in dem vorderen Ende der Mullkappe verborgen ist. Dieser Kopf gibt der Mullkappe an ihrem vorderen Ende eine gewisse Steifigkeit, so daß nicht nur ein Aufsaugen möglich ist, sondern die Mullkappe kann auch zum Reparieren von Gewebe verwendet werden, weil sie eine gewisse mechanische Steifigkeit aufweist.

In einer bevorzugten Ausgestaltung ist der Stützkörper mittels einer bevorzugt lösbaren Verrastung innerhalb des Zylinderkörpers befestigt, wobei diese Verrastung vorzugsweise an der Verbindungswand zwischen Zylinderkörper und Halterungsschaft vorgesehen ist. Die Verrastung wird hierbei über ein federndes Rastelement am freien, proximalen Ende des Stützkörpers erreicht, welches Rastelement radial federnd in eine zugeordnete Öffnung an der Verbindungswand zwischen Zylinderkörper und Halterungsschaft eingreift. Die Öffnung kann hierbei gleichzeitig zur Entlüftung des Zylinderkörpers dienen, hierzu können aber auch separat in der Verbindungswand zwischen Zylinderkörper und Halterungsschaft angeordnete Öffnungen vorgesehen sein, oder aber beide Öffnungsvarianten.

Selbstverständlich kann die Verrastung auch radial am Innenumfang der Zylinderkammer vorgesehen sein und die federnde Wirkung der Verrastung auch durch die Öffnung selbst und nicht durch die Rastelement verwirklicht werden.

Der Stützkörper ist in einer weiteren Ausgestaltung fest am Innenumfang der Zylinderkammer angeformt, wobei entsprechende Verbindungsmittel vorgesehen werden können. Eine derartige Verbindung kann z. B. sein, indem die aus Kunststoff bestehende Zylinderkammer mit einer Hitzebehandlung am Ort des Stützkörpers radial einwärts gerichtet verformt wird, so daß der Stützkörper in der Zylinderkammer, in der Art einer Prägung aufgenommen wird und damit gegen axiale Verschiebung gesichert ist.

Statt einer Verformung der Zylinderkammer mittels einem Hitzeelement kann auch eine Verschweißung des Stützkörpers am Innenumfang der Zylinderkammer stattfinden.

Der Stützkörper weist im übrigen ein oder mehrere Durchströmungsöffnungen auf, durch welche das durch die Mullkappe hindurch gesaugte Blut oder die Gewebsflüssigkeit hindurch in die Zylinderkammer strömen kann.

Das gesamte Instrument besteht bevorzugt aus einem durchscheinenden Material, so daß insbesondere im Bereich der Zylinderkammer die dort befindliche Flüssigkeit überprüft werden kann.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung, offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von lediglich einem Ausführungsweg der darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
Figur 1: Explosionsartige Darstellung einer ersten Ausführungsform des Instrumentes nach der Erfindung;
Figur 2: Das zusammengebaute Instrument nach Figur 1;
Figur 3: Schnitt entlang der Linie IV-IV, durch das zusammengebaute Instrument nach Fig. 2;
Figur 4: Schnitt entlang der Linie III-III, durch das zusammengebaute Instrument nach Fig. 2 bzw. ein um 90° gedrehter Schnitt im Vergleich zu Fig.3;
Figur 5: Explosionsartige Darstellung einer zweiten Ausführungsform des Instrumentes nach der Erfindung;
Figur 6: Das zusammengebaute Instrument nach Figur 5;
Figur 7: Schnitt entlang der Linie VIII-VIII, durch das zusammengebaute Instrument nach Fig. 6;
Figur 8: Schnitt entlang der Linie VII-VII, durch das zusammengebaute Instrument nach Fig. 6 bzw. ein um 90° gedrehter Schnitt im Vergleich zu Fig.7;

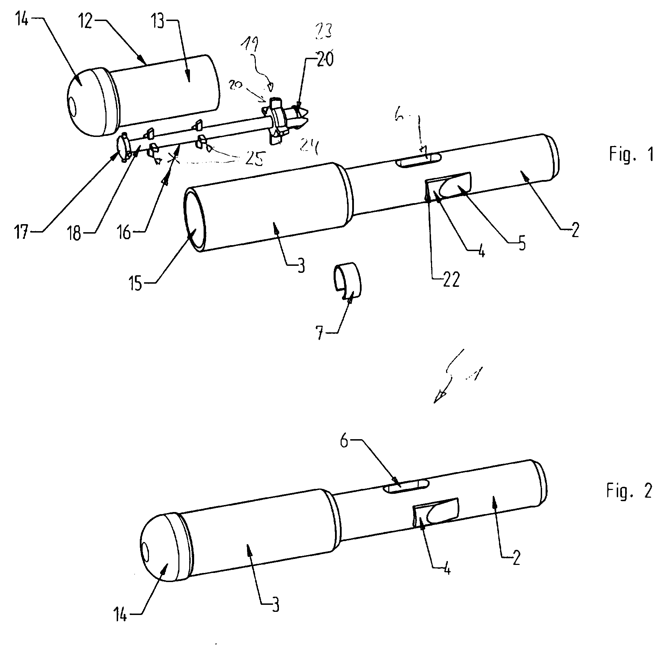

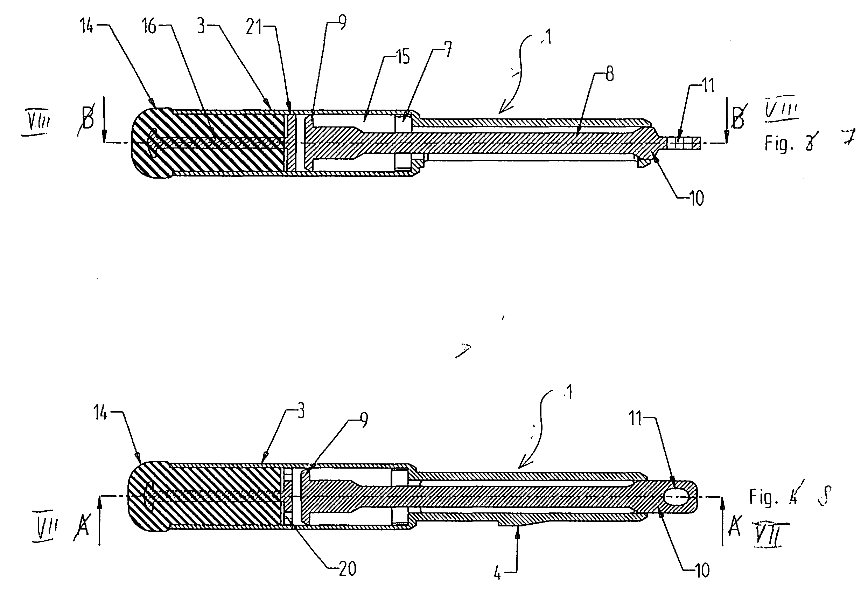

Im folgenden wir mit Bezug auf die Figuren 1-4 eine erste Ausführungsform der Erfindung beschrieben, wobei diese im Vergleich mit der zweiten Ausführungsform gemäß den Figuren 5-8 ohne eine Einrichtung zur zusätzlichen Unterdruckerzeugung innerhalb der Zylinderkammer 15 auskommt. Die absaugende Wirkung des Tupfers basiert hier also lediglich auf dem im Operationsbereich im Körper herrschenden Überdruckes

Der kleinere Zylinder des Grundkörpers 1 ist als Halterungsschaft 2 ausgebildet, der geeignet ist, in eine zugeordnete Aufnahmeöffnung eines nicht näher dargestellten Applikationsrohres, eines endoskopischen Applikators eingesteckt und dort gehalten zu werden.

Es ist nicht dargestellt, daß in diesem Applikationsrohr ein Rastfenster vorhanden ist, welches etwas größer ausgebildet ist, als die am Halterungsschaft angeordnete Rastnase, so daß beim Einstecken des Halterungsschaftes 2 in dieses Applikationsrohr die Rastnase 4 in das Rastfenster eingreift und dort mit einer Rastkante 21 einrastet.

Der Halterungsschaft 2 ist damit gegen unbeabsichtigtes Herausziehen aus dem Applikationsrohr des endoskopischen Applikators geschützt.

An dem Halterungsschaft 2 schließt sich werkstoffeinstückig ein Zylinder größeren Durchmessers an, der als Zylinderkörper 3 ausgebildet ist und der eine Zylinderkammer 15 aufweist.

Am proximalen Ende dieses Halterungsschaftes 2 ist eine Öse 26 vorgesehen, in welche ein hakenförmiges Teil eingreift, welches mit einer Zugstange fest verbunden ist, die in dem nicht näher dargestellten endoskopischen Applikator in dem vorher erwähnten Applikationsrohr längsverschieblich geführt ist und somit das Instrument axial bewegt werden kann.

Zum erleichternden Einrasten der Rastnase 4 in das nicht näher dargestellte Rastfenster des Applikationsrohres weist die Rastnase 4 an ihrem proximalen Ende eine Anschrägung 5 auf, die als Gleitfläche ausgebildet ist, so daß also zunächst die Gleitfläche auf den Innenumfang des Applikationsrohres trifft, hierbei die Rastnase 4 radial einwärts nach innen verformt wird und sobald die Rastnase 4 in Gegenüberstellung zu dem nicht näher dargestellten Rastfenster gelangt, rastet diese aus und die Rastkante 22 legt sich an einer zugeordneten Kante des Rastfensters an. Zur Betätigung der Rastnase 4 ist im übrigen ein Schlitz 6 im Halterungsschaft 2 angeordnet.

Das Instrument 1 nach der Erfindung besteht im wesentlichen aus einem doppelten Hohlzylinder, wobei ein vorderer Zylinderkörper 3 werkstoffeinstückig mit einem Halterungsschaft 2 geringeren Durchmessers verbunden ist.

Wie angegeben, ist mindestens der Zylinderkörper 3 aus einem durchscheinenden Material gefertigt; aus Vereinfachungsgründen ist aber das Instrument 1 insgesamt durchscheinend ausgebildet.

Wichtig ist nun, daß am vorderen Ende der Zylinderkammer 15 eine Mullkappe 12 fest befestigt ist. Die Befestigung erfolgt hierbei mittels eines Stützkörpers 16. Die Mullkappe 12 ist hierbei auf dem Stützkörper 16 aufgewickelt, damit sich so ein Schaft 13 geringeren Durchmessers bildet, der bündig in die Zylinderkammer 15 hineinpaßt, während das vordere Ende des Schaftes 13 durch einen Kopf 14 größeren Durchmessers abgeschlossen ist.

Hierbei ist die Mullkappe 12 auf einem Stützkörper 16 angeordnet und der Stützkörper 16 selbst wird mittels einer Verrastung 23 in Form eines zweigeteilten, widerhakenartigen Ansatzes eingerastet.

Der Stützkörper 16 besteht im wesentlichen aus einer Scheibe 19 größeren Durchmessers, auf welchem radial Ansätze 24 angebracht sind, wodurch Ausnehmungen 20 gebildet werden, die eventuell eingeschlossene Luft durch eine hintere Bohrung im Instrument 1 entweichen läßt und hierbei die Luft auch durch die mittlere Öffnung entweicht, durch welche die widerhakenartigen Ansätze 23 des Stützkörpers 16 greifen.

Der Außendurchmesser der Scheibe 19 mit den daran angebrachten radialen Ansätzen 24 entspricht etwa dann dem Innendurchmessers der Zylinderkammer 15. Die Scheibe 19 ist mit einer Stange 18 verbunden, die einen vorderen Kopf 17 größeren Durchmessers aufweist. Auf der Stange 18 sind weitere radiale Ansätze 25 vorgesehen, welche geringere radiale Abmessungen aufweisen, wie der Durchmesser der Zylinderkammer 15 und welche das axiale Verrutschen der Mullkappe 12 verhindern sollen. Einige dieser radialen Ansätze 25 sind hierbei innerhalb der Mullkappe 12 vorgesehen, andere an der Stirnseite der Mullkappe 12.

In einer anderen Ausgestaltung der Erfindung kann auf den Ansätzen 24 der Scheibe 19 umfangsseitig ein Ring 7 aufgebracht sein, welcher etwa dem Innendurchmesser der Zylinderkammer 15 entspricht. Der Ring 7 besteht aus einem röntgendichten Material, wobei der Sinn dieses Ringes darin liegt, daß wenn unerwarteter Weise das Instrument 1 im Körper verloren geht, das es dann durch eine Röntgenaufnahme lokalisierbar ist. Dieser Ring muß aber nicht zwingend auf der Scheibe 19 aufgebracht sein, sondern kann beispielsweise auch außerhalb des Bereiches der Scheibe 19 am Innenumfang der Zylinderkammer 15 angebracht sein, jedoch wird es möglichst vermieden diesen Ring 7 außen am Instrument 1 anzubringen, da er verloren gehen könnte.

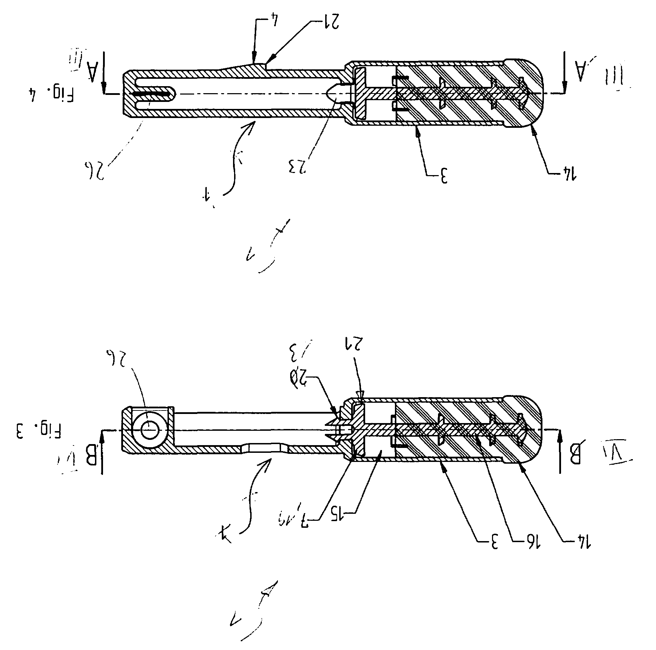

Nachdem Aufwickeln der Mullkappe 12 auf den Stützkörper 16 wird dieser in der Art nach Figur 3 und 4 in die Zylinderkammer 15 eingeführt. Der Rand 21 der Scheibe 19 liegt dann am Innenumfang der Zylinderkammer 15 an und wird über die Verrastung 23 gegen axiale Verschiebung gesichert.

Im praktischen Einsatz dieses Instrumentes 1 wird dieses in ansich bekannter Weise in die Nähe des Operationsgebietes gebracht, wobei der Kolben 9 in seiner ausgeschobenen, vorne liegenden Position ist, wie dies in den Figuren 3 und 4 dargestellt ist.

Soll nun Blut oder Körperflüssigkeit aufgenommen werden, dann wird die Mullkappe 12 in die Körperflüssigkeit eingetaucht und hierdurch das Blut oder die Gewebsflüssigkeit durch die Mullkappe 12 hindurch in die Zylinderkammer 15 eingsaugt.

Wenn die Zylinderkammer 15 vollgefüllt ist und die Mullkappe 12 kein weiteres Aufsaugvermögen mehr hat, kann das gesamte Instrument 1 zusammen mit dem Applikator aus dem Operationsgebiet entfernt werden.

Im folgenden wir mit Bezug auf die Figuren 5-8 die zweite Ausführungsform der vorliegenden Erfindung näher beschrieben, wobei hier im Gegensatz zur oben beschriebenen ersten Ausführungsform ein zusätzlicher Unterdruck in der Zylinderkammer 15 durch einen Kolben 9 zur Erhöhung des Absaug-Wirkungsgrades erzeugt wird.

Es ist also innerhalb des Instrumentes 1 ein Kolben 9 mit einer daran angebrachten Kolbenstange 8 vorgesehen. Der Stützkörper 16 ist hierbei etwas andersartig ausgebildet, er besitzt nämlich nicht die oben beschriebene Verrastung 23, sondern ist frei über den Scheibenrand 21 innerhalb der Zylinderkammer 15 gelagert.

Die prinzipielle Funktionsweise des Instruments der zweiten Ausführungsform ist etwa die gleich wie die bei dem Instrument der ersten, oben beschriebenen Ausführungsform, wobei alle gleich bezeichneten Bauteile die gleiche Funktion besitzen.

Insbesondere ist der Grundkörper 1 mit dem Halterungsschaft 2 und dem Zylinderkörper 3 mit Zylinderkammer 15 gleich aufgebaut wie in den Figuren 1 bis 4 und die Kopplung des kleineren Zylinder des Grundkörpers 1 ist ebenfalls geeignet, in eine zugeordnete Aufnahmeöffnung eines nicht näher dargestellten Applikationsrohres, eines endoskopischen Applikators eingesteckt und dort gehalten zu werden.

Auch die Funktion der am Halterungsschaft angeordneten Rastnase 4 und des Schlitzes 6 entspricht der von Figur 1 bis 4.

In der Zylinderkammer 15 des Zylinderkörpers 3 ist und nun in dieser Ausführung ein Kolben 9 längsverschieblich eingebracht, der fest mit einer Kolbenstange 8 verbunden ist.

Die Kolbenstange 8 greift also in den Zylinderkörper 3 hinein, greift durch den Halterungsschaft 2 hindurch und ragt auf dem hinteren Ende des Halterungsschaftes 2 in Form einer Einhängung 10 heraus, welche mit einer Öse 11 verbunden ist.

In diese Öse 11 greift ein hakenförmiges Teil, welches mit einer Zugstange fest verbunden ist, die in dem nicht näher dargestellten endoskopischen Applikator in dem vorher erwähnten Applikationsrohr längsverschieblich geführt ist.

Mit der Betätigung der Zugstange, wird damit die Kolbenstange 8 in dem Grundkörper 1 längsverschieblich verschoben und der Kolben 9 gleitet damit in der Zylinderkammer 15 hin und her.

In den beiden Zylinderkörpern 2, 3 ist die Kolbenstange 8 längsverschiebbar geführt, die einen vorderen Kolben 9 aufweist, der abdichtend am Innenumfang der Zylinderkammer 15 verschiebbar ausgebildet ist. Die Verschiebung der Kolbenstange 8 erfolgt hierbei dadurch, daß in einer hinteren Einhängung 10 eine Öse 11 eingearbeitet ist, die mit einem nicht näher dargestellten Haken einer Zugstange zusammenwirkt, die in einem Applikationsrohr eines endoskopischen Applikators längs verschiebbar geführt ist.

Wichtig ist nun, daß auch in dieser zweiten Ausbildung der Erfindung, am vorderen Ende der Zylinderkammer 15 eine Mullkappe 12 fest befestigt ist. Die Befestigung erfolgt hierbei mittels eines Stützkörpers 16. Die Mullkappe 12 ist hierbei auf dem Stützkörper 16 aufgewickelt, damit sich so ein Schaft 13 geringeren Durchmessers bildet, der bündig in die Zylinderkammer 15 hineinpaßt, während das vordere Ende des Schaftes 13 durch einen Kopf 14 größeren Durchmessers abgeschlossen ist.

Der Stützkörper 16 besteht im wesentlichen aus einer Scheibe 19 größeren Durchmessers, deren Außendurchmesser etwa dem Innendurchmessers der Zylinderkammer 15 besteht, wobei die Scheibe 19 ein oder mehrere Ausnehmungen 20 aufweist. Die Scheibe 19 ist mit einer Stange 18 verbunden, die einen vorderen Kopf 17 größeren Durchmessers aufweist.

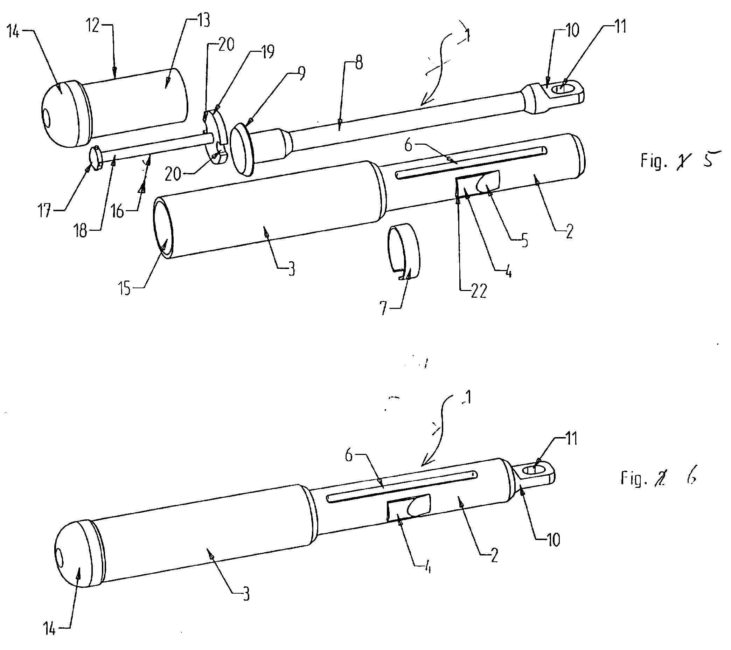

Nachdem Aufwickeln der Mullkappe 12 auf den Stützkörper 16 wird dieser in der Art nach Figur 7 und 8 in die Zylinderkammer 15 eingeführt. Der Rand 21 der Scheibe 19 liegt dann am Innenumfang der Zylinderkammer 15 an und wird dort gegen axiale Verschiebung gesichert festgelegt.

Wie im allgemeinen Beschreibungsteil angegeben, erfolgt diese Sicherung beispielsweise dadurch, daß die Zylinderkammer 15 am Ort dieses Randes 21 mit einem Hitzeinstrument so verformt wird, daß sie sich radial einwärts zieht und damit eine Einprägung bildet, die damit die Scheibe 19 gegen axiale Verschiebung in der Zylinderkammer 15 sichert.

Selbstverständlich kann auch der Rand der Scheibe 21 mittels einer Ultraschallschweißung oder Klebung, oder einem anderen Befestigungsmittel fest mit dem Innenumfang der Zylinderkammer 15 verbunden werden.

Im praktischen Einsatz dieses Instrumentes 1 wird dieses in ansich bekannter Weise in die Nähe des Operationsgebietes gebracht, wobei der Kolben 9 in seiner ausgeschobenen, vorne liegenden Position ist, wie dies in den Figuren 7 und 8 dargestellt ist.

Soll nun Blut oder Körperflüssigkeit aufgenommen werden, dann wird die Mullkappe 12 in die Körperflüssigkeit eingetaucht und gleichzeitig wird mittels der Zugstange des Applikator die Kolbenstange 8 nach hinten gezogen, wodurch ein Vakuum im vorderen Teil der Zylinderkammer 15 direkt hinter der Scheibe 19 des Stützkörpers 16 erzeugt wird, und hierdurch das Blut oder die Gewebsflüssigkeit durch die Mullkappe 12 hindurch in die Zylinderkammer 15 eingsaugt wird.

Wenn die Zylinderkammer 15 vollgefüllt ist und die Mullkappe 12 kein weiteres Aufsaugvermögen mehr hat, kann das gesamte Instrument 1 zusammen mit dem Applikator aus dem Operationsgebiet entfernt werden.

Auch hier ist wieder ein Ring 7 aus einem röntgendichten Material vorgesehen, der am Innenumfang der Zylinderkammer 15 eingesetzt wird.

### Zeichnungslegende

- 1.: Instrument
- 2.: Halterungsschaft
- 3.: Zylinderkörper
- 4.: Rastnase
- 5.: Anschrägung
- 6.: Schlitz
- 7.: Ring
- 8.: Kolbenstange
- 9.: Kolben
- 10.: Einhängung
- 11.: Öse
- 12.: Mullkappe
- 13.: Schaft
- 14.: Kopf
- 15.: Zylinderkammer
- 16.: Stützkörper
- 17.: Kopf
- 18.: Stange
- 19.: Scheibe
- 20.: Ausnehmung
- 21.: Scheibenrand
- 22.: Rastkante
- 23.: Verrastung
- 24.: Fuß
- 25.: Ansätze
- 26.: Öse

## Patentansprüche

1. Endkoskopisches Instrument zur Präparation bei mikrochirurgischen Operationen und Untersuchungen **dadurch gekennzeichnet,** daß eine als Tupfer ausgebildete Mullkappe (12) am vorderen, freien Ende einer Zylinderkammer (15) angeordnet ist.

2. Endkoskopisches Instrument zur Präparation nach Anspruch 1, **dadurch gekennzeichnet,** daß die Mullkappe (12) auf ein Stützelement (16) aufgeformt ist.

3. Endkoskopisches Instrument zur Präparation nach Anspruch 2, **dadurch gekennzeichnet,** daß der Stützkörper (16) mittels einer widerhakenartigen Verrastung (23) am Instrument (1) eingerastet wird.

4. Endkoskopisches Instrument zur Präparation nach Anspruch 3, **dadurch gekennzeichnet,** daß die Verrastung (23) lösbar vorgesehen ist.

5. Endkoskopisches Instrument zur Präparation nach einem der Ansprüche 3 oder 4, **dadurch gekennzeichnet,** daß die Verrastung (23) durch das Eingreifen eines im wesentlichen radial elastisch federnden Ansatzes auf der proximalen Stirnseite der Scheibe (19) in eine Öffnung in der Trennwand zwischen Zylinderkammer (15) und Halterungsschaft (2) erfolgt.

6. Endkoskopisches Instrument zur Präparation nach Anspruch 2, **dadurch gekennzeichnet,** daß in der Zylinderkammer (15) ein Kolben (9) abdichtend, längsverschiebbar geführt ist und dieser Kolben (9) beim Zurückziehen einen Unterdruck auf die am distalen Ende angeordnete Mullkappe (12) ausübt.

7. Endkoskopisches Instrument zur Präparation nach einem der Ansprüche 1 bis 6, **dadurch gekennzeichnet,** daß das endoskopische Instrument (1) leicht auswechselbar am vorderen freien Ende eines endoskopischen Applikationsrohres angeordnet ist, welches seinerseits fest mit einem endoskopischen Applikator verbunden ist.

8. Endkoskopisches Instrument zur Präparation nach Anspruch 7, **dadurch gekennzeichnet,** daß die Befestigung des endoskopischen Instrumentes am vorderen freien Ende des endoskopischen Applikationsrohres mittels einer Rastbesfestigung oder einer Schraubverbindung oder einer Bajonettsteckverbindung bewerkstelligt wird.

9. Endkoskopisches Instrument zur Präparation nach einem der Ansprüche 2 bis 8, **dadurch gekennzeichnet,** daß das Stützelement (16) ein oder mehrere in axialer Richtung sich erweiternde Vorsprünge (17, 19; 17, 19, 24, 25) aufweist.

10. Endkoskopisches Instrument zur Präparation nach einem der Ansprüche 2 bis 9, **dadurch gekennzeichnet,** daß das Stützelement (16) an seinem distalen Ende mit einem Kopf (17) ausgebildet ist, der in dem vorderen Ende der Mullkappe (12) verborgen ist.

11. Endkoskopisches Instrument zur Präparation nach einem der Ansprüche 2 bis 10, **dadurch gekennzeichnet,** daß das Stützelement (16) an seinem proximalen Ende mit einer Scheibe (19) ausgebildet ist, welche mindestens eine Ausnehmung (20) besitzt, die den Raum vor und hinter der Stirnseite der Scheibe (19) miteinander verbindet.

12. Endkoskopisches Instrument zur Präparation nach Anspruch 11, **dadurch gekennzeichnet,** daß die Ausnehmungen (20) durch Bohrungen oder radiale Ansätze (24) am Scheibenrand (21) gebildet werden.

13. Endkoskopisches Instrument zur Präparation nach einem der Ansprüche 2 bis 12, **dadurch gekennzeichnet,** daß das Stützelement (16) über optionale Verbindungsmittel am Innenumfang der Zylinderkammer (15) angeformt ist.

14. Endkoskopisches Instrument zur Präparation nach einem der Ansprüche 1 bis 13, **dadurch gekennzeichnet,** daß die Zylinderkammer (15) aus Kunststoff besteht.

15. Endkoskopisches Instrument zur Präparation nach Anspruch 14, **dadurch gekennzeichnet,** daß die Verbindung zwischen Stützelement (16) und Kunststoff-Zylinderkammer (15) durch Verschweißung oder Wärmeverformung der Zylinderkammer (15) radial nach innen erreicht wird.

16. Endkoskopisches Instrument zur Präparation nach einem der Ansprüche 2 - 15, **dadurch gekennzeichnet,** daß das Stützelement (16) und die Zylinderkammer (15) im wesentlichen keine Relativbewegung mindestens in axialer Richtung zueinander ausführen.

17. Endkoskopisches Instrument zur Präparation nach einem der Ansprüche 1 bis 16, **dadurch gekennzeichnet,** daß mindestens die Zylinderkammer (15) teilweise derart transparent ausgebildet ist, daß die dort befindliche Flüssigkeit optisch überprüft werden kann.
